# EUROPEAN PATENT SPECIFICATION

(11) **EP 2 638 846 B1**
(45) Date of publication and mention of the grant of the patent: **02.03.2016**
(21) Application number: 12792189.8
(22) Date of filing: 05.03.2012
(51) Int. Cl.: A61B 1/04, G01N 21/64, A61B 1/00, A61B 1/045

(54) **MEDICAL APPARATUS**
MEDIZINISCHE VORRICHTUNG
DISPOSITIF MÉDICAL

(30) Priority: 27.05.2011 JP 2011119558
(43) Date of publication of application: 18.09.2013
(73) Proprietor: Olympus Corporation, Shibuya-ku Tokyo (JP)
(72) Inventor: TAKEI, Shunji, Tokyo 151-0072 (JP); DOGUCHI, Nobuyuki, Tokyo 151-0072 (JP)
(74) Representative: Gunzelmann, Rainer
(86) International application number: PCT/JP2012/055560
(87) International publication number: WO 2012/164991

(56) References cited:
- EP-A2- 2 123 213
- JP-A- 2004 024 611
- JP-A- 2005 329 115
- JP-A- 2006 175 052
- JP-A- 2007 075 198
- JP-A- 2010 516 332
- US-A1- 2002 138 008
- US-A1- 2005 154 319
- US-A1- 2010 245 616

## Description

### Technical Field

The present invention relates to a medical apparatus, and particularly relates to a medical apparatus capable of performing fluorescence observation.

### Background Art

As a method for finding a site where a lesion such as an early cancer is present, for example, a method has been studied in recent years, which uses a fact that when a site to be observed of a living body is irradiated with an excitation light, intensity of fluorescence which is emitted from a normal tissue and intensity of fluorescence which is emitted from a lesioned tissue differ from each other.

For example, as a configuration for realizing the aforementioned method, Japanese Patent Application Laid-Open Publication No. 2005-329115 discloses a fluorescence endoscope apparatus that generates a synthesized image in which an image signal of a reflected light image that is a result of picking up of images of reflected lights in a living tissue by two illuminating lights of narrow band, and an image signal of a fluorescence image that is a result of picking up of an image of fluorescence generated from the living tissue in response to irradiation of an excitation light of a narrow band are synthesized.

Further, for example, Japanese Patent Application Laid-Open Publication No. 2001-258820 discloses a fluorescence endoscope apparatus that performs pixel binning (binning reading) in an image pickup device, as a configuration for increasing received light amount per one pixel of the fluorescence emanated from a lesioned tissue.

However, in a case where the pixel binning as disclosed in above-mentioned Japanese Patent Application Laid-Open Publication No. 2001-258820 is applied to be simply in combination when generating a synthesized image such as disclosed in above-mentioned Japanese Patent Application Laid-Open Publication No. 2005-329115 , there arises a problem of generating a synthesized image with a lowered resolution in which, for example, information useful for diagnosis of a site to be observed is largely missing.
On the other hand, depending on whether the pixel binning as disclosed in above-mentioned Japanese Patent Application Laid-Open Publication No. 2001-258820 is carried out or not, a light amount of the fluorescence received by the image pickup device is sharply increased of decreased. Therefore, in the case where the pixel binning as disclosed in above-mentioned Japanese Patent Application Laid-Open Publication No. 2001-258820 is applied to be simply in combination when generating a synthesized image such as disclosed in above-mentioned Japanese Patent Application Laid-Open Publication No. 2005-329115 , there arises a problem of generating a synthesized image with brightness unsuitable for observation in which, for example, brightness of a fluorescence image with respect to a reference light image is excessive or lacking.

US 2002/0138008 A1 discloses a method and an apparatus for displaying a fluorescence image in which operation processing is performed on a first fluorescence image and at least either one of a second fluorescence image and a reflected reference light image to form a tissue condition image, which represents a tissue condition of living body tissues and has been compensated for a distance to the living body tissues. According to at least one of the three images, a judgment is made as to whether each image area in the tissue condition image is an abnormal light affected area or a normal light detection area. The abnormal light affected area is displayed in a form different from the normal light detection area.

The present invention has been made in view of the above circumstances, and an object of the present invention is to provide a medical apparatus capable of generating an observed image by image synthesis to have brightness and resolution suitable for fluorescent observation.

### Disclosure of Invention

### Means for Solving the Problem

A medical apparatus of one aspect of the present invention comprises the features of claim 1.

### Brief Description of the Drawings

Fig. 1 is a diagram showing a configuration of an essential part of an endoscope apparatus according to an embodiment of the present invention;
Fig. 2 is a flowchart showing one example of processing and the like which are performed in a fluorescence observation mode; and
Fig. 3 is a flowchart showing an example of processing and the like which are performed in the fluorescence observation mode, which is different from Fig. 2.

### Best Mode for Carrying Out the Invention

Hereinafter, an embodiment of the present invention will be described with reference to the drawings.

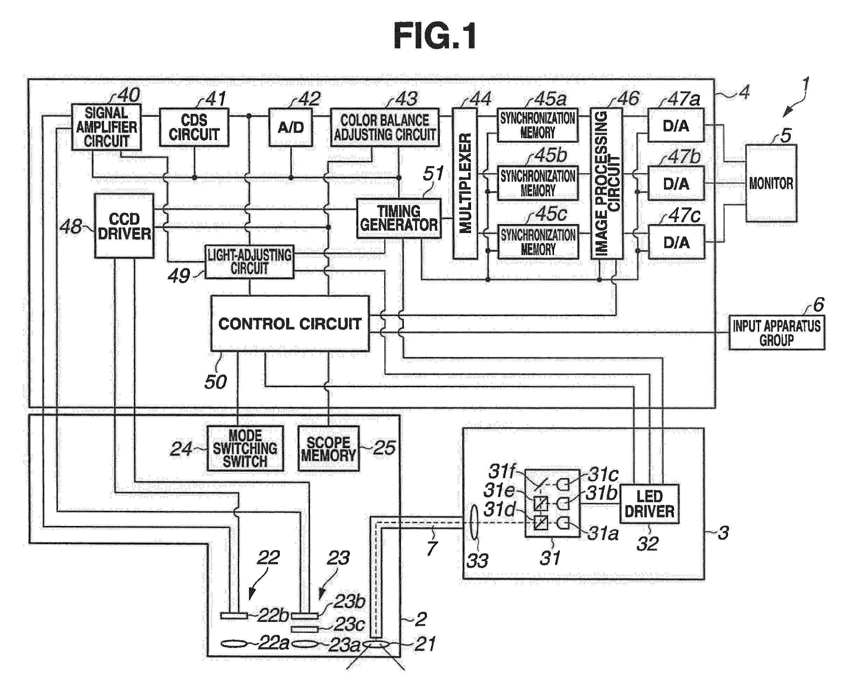

Fig. 1 is a diagram showing a configuration of an essential part of an endoscope apparatus according to an embodiment of the present invention.

As shown in Fig. 1, an endoscope apparatus 1 is configured by having an endoscope 2 which is insertable into a subject that is a living body, acquires an image of an object such as a living tissue existing in the subject, and outputs an acquired image of the object as an image pickup signal, a light source apparatus 3 that supplies a light, which is emitted to the object, to the endoscope 2 via a light guide 7 that is inserted through an interior of the endoscope 2, a processor 4 which performs signal processing corresponding to an image pickup signal which is outputted from the endoscope 2, and outputs an image pickup signal after the signal processing is performed therefor as a video signal, a monitor 5 which performs image display of the image of the object which is acquired in the endoscope 2 based on the video signal outputted from the processor 4, and an input apparatus group 6 including a switch, buttons and the like capable of input operation to the processor 4.

The endoscope 2 is configured by having an illumination optical system 21 which emits light that is supplied from the light source apparatus 3 and is transmitted by the light guide 7 to an object, a white color light observing image pickup section 22, a fluorescence observing image pickup section 23, a mode switching switch 24 capable of performing an operation relating to switching of an observation mode of the endoscope apparatus 1, and a scope memory 25 storing information peculiar to the endoscope 2.

The white color light observing image pickup section 22 is configured by having an objective optical system 22a which forms an image of an object, and a CCD 22b with an image pickup surface that includes a primary color filter being disposed to correspond to an image forming position of the objective optical system 22a.

The CCD 22b drives in response to control of the processor 4, and generates an image pickup signal by applying photoelectric conversion to a return light from the object an image of which is formed on the image pickup surface, and outputs the image pickup signal to the processor 4.

The fluorescence observing image pickup section 23 is configured by having an objective optical system 23 a that forms an image of an object, a monochrome CCD 23b with an image pickup surface being disposed to correspond to an image forming position of the objective optical system 23a, and an excitation light cut filter 23c which is disposed at a front stage of the CCD 23b.

The CCD 23b drives in response to control of the processor 4, and generates an image pickup signal by applying photoelectric conversion to a return light from an object an image of which is formed on the image pickup surface, and outputs the image pickup signal to the processor 4.

Further, the CCD 23b is configured by including a function as a pixel addition processing section. More specifically, the CCD 23b is configured by having a pixel binning function that is a function of being able to output a signal which is a result of addition processing (pixel addition processing) of making signals corresponding to a plurality of pixels a signal corresponding to one pixel.

The excitation light cut filter 23c is formed by including such optical characteristics that shut off a wavelength band of an excitation light which is emanated from an excitation light LED 31b which will be described later, and transmit a wavelength band of fluorescence that is emanated from a fluorescent substance such as a fluorescent agent that is excited by the excitation light, and a wavelength band of a reference light that is emanated from a reference light LED 31c which will be described later respectively.

The scope memory 25 previously stores information including a number of added pixels PA at a time of an operation of the pixel binning function of the CCD 23b, and a target value α of a brightness ratio of a fluorescence image and a reference light image which are picked up by the fluorescence observing image pickup section 23.

More specifically, the aforementioned number of added pixels PA is set as, for example, a number of pixels which is obtained by calculation of a predicted value of an intensity of fluorescence that is emanated from an applied site based on fluorescent characteristics of a fluorescent agent which is given to the applied site of the endoscope 2, and optimization being performed with respect to the calculated predicted value.

Further, the aforementioned target value α of the brightness ratio is set as a value of a ratio showing a degree of a brightness of the fluorescence image acquired by the CCD 23b when the aforementioned reference object is irradiated with an excitation light in a case in which a brightness of a reference light image acquired by the CCD 23b when the reference object including substantially ideal fluorescent characteristics and reflection characteristics is irradiated with a reference light, for example.

Note that the scope memory 25 is not limited to the scope memory which stores only one kind of the number of added pixels PA and the target value α of the brightness ratio which are set in accordance with the applied site of the endoscope 2, but the scope memory 25 may store a plurality of kinds of numbers of added pixels and target values of the brightness ratio which are set for each of the respective sites which can be the applied targets of the endoscope 2, in a form of table data or the like, for example.

The light source apparatus 3 has an LED light source section 31, an LED driver 32, and a condensing optical system 33 which condenses light emanated in the LED light source section 31 and supplies the light to the light guide 7.

The LED light source section 31 is configured by having a white color light LED 31a, an excitation light LED 31b, a reference light LED 31c, a half mirror 31d, a half mirror 31e and a mirror 31f.

The white color light LED 31a is configured to be able to emanate a white color light including respective wavelength bands of R (red color), G (green color) and B (blue color).

The excitation light LED 31b is configured to be able to emanate an excitation light of a wavelength band capable of exciting a fluorescent substance such as a fluorescent agent.

The reference light LED 31c is configured to be able to emanate a reference light of a wavelength band which does not overlap the wavelength band of the excitation light emanated from the excitation light LED 31b, and does not overlap the wavelength band of the fluorescence emanated from the fluorescent substance such as a fluorescent agent that is excited by the excitation light.

The half mirror 31d is disposed on an optical path between the white color light LED 31a and the condensing optical system 33, and is configured by including such optical characteristics that transmit the white color light emanated from the white color light LED 31a to a condensing optical system 33 side, and reflect the excitation light and the reference light which are emitted via the half mirror 31e to the condensing optical system 33 side.

The half mirror 31e is disposed on an optical path between the half mirror 31d and the mirror 31f, and is configured by including such optical characteristics that reflect the excitation light emanated from the excitation light LED 31b to a half mirror 31d side, and transmit the reference light emitted via the mirror 31f to the half mirror 31 d side.

The mirror 31f is configured by including such optical characteristics that reflect the reference light emanated from the reference light LED 31c to a half mirror 31e side.

The LED driver 32 is configured to be able to supply a drive electric current for driving the respective LEDs which are provided in the LED light source section 31. Therefore, for example, with the magnitude of the drive electric current which is supplied to the LED light source section 31 from the LED driver 32 changing, the intensity of the lights (the white color light, the excitation light and the reference light) emanated from the LED light source section 31 changes.

Also, the LED driver 32 operates to cause the respective LEDs provided in the LED light source section 31 to emit lights or quench lights in response to control of the processor 4.

More specifically, when the LED driver 32 detects that the endoscope apparatus 1 is switched to a white color light observation mode, based on a mode switching control signal outputted from a control circuit 50 of the processor 4, the LED driver 32 operates to cause the white color light LED 31a to emit a light and cause the excitation light LED 31b and the reference light LED 31c to quench lights. Further, when the LED driver 32 detects that the endoscope apparatus 1 is switched to a fluorescence observation mode, based on the mode switching control signal outputted from the control circuit 50 of the processor 4, the LED driver 32 operates to cause the white color light LED 31a to quench a light, and cause the excitation light LED 31b and the reference light LED 31c to emit lights alternately.

Also, in the case in which the endoscope apparatus 1 is switched to the fluorescence observation mode, the LED driver 32 generates identifying signals respectively capable of identifying time periods in which the excitation light LED 31b is caused to emit a light and quench a light, and time periods in which the reference light LED 31c is caused to emit a light and quench a light, and outputs the identifying signals to a timing generator 51.

According to the present embodiment, in place of the light source apparatus 3 having the configuration described above, a light source apparatus of a known configuration can be used, which includes, for example, a lamp that emanates a white color light, and a rotary filter that has a filter group for white color light observation and a filter group for fluorescence observation, and capable of disposing one of the filter groups on an optical path of the lamp in response to switching of the observation mode.

The processor 4 has a signal amplifier circuit 40 that performs processing relating to amplification of a signal, a CDS circuit 41 that performs CDS (correlated double sampling) processing, an A/D converting circuit 42 that performs A/D conversion processing, a color balance adjusting circuit 43 that performs color balance adjustment processing, a multiplexer 44 that performs an operation relating to distribution of signals, synchronization memories 45a, 45b and 45c, an image processing circuit 46 that performs predetermined image processing, and D/A converting circuits 47a, 47b and 47c that perform D/A conversion processing.

Further, the processor 4 is configured by having a CCD driver 48 that controls drive states of the CCDs 22b and 23b in response to switching of the observation mode of the endoscope apparatus 1, a light-adjusting circuit 49 that performs an operation relating to adjustment of brightness of an image, the control circuit 50 that performs various controls corresponding to switching of the observation mode of the endoscope apparatus 1, and the timing generator 51 that performs an operation relating to generation of a timing signal.

Here, the image pickup signal which is outputted from the CCD 22b in the white color light observation mode, and the image pickup signal which is outputted from the CCD 23b in the fluorescence observation mode are both inputted in the signal amplifier circuit 40 of the processor 4.

The signal amplifier circuit 40 is configured by an AGC (automatic gain control) circuit and the like, and based on the timing signal from the timing generator 51, the signal amplifier circuit 40 performs signal amplification corresponding to control of the light-adjusting circuit 49 for an image pickup signal which is inputted in the processor 4 and outputs the image pickup signal to the CDS circuit 41.

Based on the timing signal from the timing generator 51, the CDS circuit 41 performs CDS processing for the image pickup signal which is outputted from the signal amplifier circuit 40 and outputs the image pickup signal to the A/D converting circuit 42 and the light-adjusting circuit 49.

The A/D converting circuit 42 generates a digital image signal by applying A/D conversion processing to the image pickup signal which is outputted from the CDS circuit 41 based on the timing signal from the timing generator 51, and outputs the image signal to the color balance adjusting circuit 43.

The color balance adjusting circuit 43 applies color balance adjustment processing to the image signal which is outputted from the A/D converting circuit 42, based on the timing signal from the timing generator 51, and outputs the image signal to the multiplexer 44.

The multiplexer 44 separates the image signal which is outputted from the color balance adjusting circuit 43 in accordance with the respective color components and outputs the separated image signals of the respective color components to the synchronization memories 45a, 45b and 45c while the multiplexer 44 distributes the image signals to the synchronization memories 45a, 45b and 45c.

The synchronization memories 45a, 45b and 45c have configurations capable of temporarily storing the image signals of the respective color components which are outputted from the multiplexer 44.

The image processing circuit 46 simultaneously reads the respective image signals stored in the synchronization memories 45a, 45b and 45c, based on the timing signal from the timing generator 51, and thereafter applies image processing such as gamma correction to the respective image signals which have been read. Subsequently, the image processing circuit 46 allocates the respective image signals to which the image processing such as gamma correction has been applied to a first color channel corresponding to a first color component (for example, an R component), a second color channel corresponding to a second color component (for example, a G component), and a third color channel corresponding to a third color component (for example, a B component) respectively, and outputs the respective image signals to the D/A converting circuits 47a, 47b and 47c.

Further, the image processing circuit 46 performs image processing corresponding to control of the control circuit 50, and also operates to generate GUI (graphical user interface) or the like corresponding to control of the control circuit 50 and display GUI or the like on the monitor 5.

In the meantime, the image signals of the first color component, the second color component and the third color component, which are outputted from the image processing circuit 46, are converted into analogue video signals respectively in the D/A converting circuits 47a, 47b and 47c, and thereafter are outputted to the monitor 5. Thereby, observed images corresponding to the respective observation modes are displayed on the monitor 5.

The CCD driver 48 operates to switch drive states of the CCD 22b and the CCD 23b in response to control of the control circuit 50.

More specifically, when the CCD driver 48 detects that the endoscope apparatus 1 is switched to the white color light observation mode, based on the mode switching control signal which is outputted from the control circuit 50, the CCD driver 48 operates to drive the CCD 22b and stop drive of the CCD 23b. Further, when the CCD driver 48 detects that the endoscope apparatus 1 is switched to the fluorescence observation mode, based on the mode switching control signal which is outputted from the control circuit 50, the CCD driver 48 operates to drive the CCD 23b and stop the drive of the CCD 22b.

Also, the CCD driver 48 operates to synchronize switching of on and off of pixel binning corresponding to control of the control circuit 50 with the timing signal from the timing generator 51, in the middle of the CCD driver 48 driving the CCD 23b.

More specifically, when the CCD driver 48 detects that it is a time period in which the excitation light LED 31b emits light based on the timing signal from the timing generator 51, in the middle of the CCD driver 48 driving the CCD 23 b, the CCD driver 48 operates so that pixel binning by the number of added pixels corresponding to the control of the control circuit 50 is implemented. Further, when the CCD driver 48 detects that it is a time period in which the reference light LED 31c emits light based on the timing signal from the timing generator 51, in the middle of the CCD driver 48 driving the CCD 23b, the CCD driver 48 operates so that pixel binning by the number of added pixels corresponding to the control of the control circuit 50 is not implemented.

The light-adjusting circuit 49 reads the target value of the brightness corresponding to the observation mode selected by operation of the mode switching switch 24 from a memory or the like not illustrated, based on the mode switching control signal which is outputted from the control circuit 50. Subsequently, based on the timing signal from the timing generator 51, the target value of the brightness which is read from the memory or the like not illustrated, and the image pickup signal which is outputted from the CDS circuit 41, the light-adjusting circuit 49 generates a light adjustment signal for adjusting the brightness of the image pickup signal and outputs the light adjustment signal to the LED driver 32 and the signal amplifier circuit 40.

The control circuit 50 performs control so as to cause the respective sections of the endoscope apparatus 1 to perform actions corresponding to the operations of the input apparatus group 6. Further, the control circuit 50 generates and outputs a mode switching control signal for causing the respective sections of the endoscope apparatus 1 to perform the actions of the observation mode selected by operation of the mode switching switch 24. Furthermore, the control circuit 50 performs control so as to cause the respective sections of the endoscope apparatus 1 to perform actions corresponding to the information read from the scope memory 25.

The timing generator 51 generates and outputs a timing signal for properly synchronizing the actions of the respective sections of the processor 4 in the case in which an identifying signal is outputted from the LED driver 32.

More specifically, in a case in which an identifying signal is outputted from the LED driver 32, that is, in a case in which the endoscope apparatus 1 is switched to the fluorescence observation mode, the timing generator 51 generates and outputs a timing signal for causing the respective sections of the processor 4 to perform the actions synchronized with the time period in which the excitation light LED 31b emits light (or quenches light) and the time period in which the reference light LED 31c emits light (or quenches light).

Note that in a case in which an identifying signal is not outputted from the LED driver 32, that is, in a case in which the endoscope apparatus 1 is switched to the white color light observation mode, the timing generator 51 does not perform generation of a timing signal. Therefore, in the case in which the endoscope apparatus 1 is switched to the white color light observation mode, synchronization of the actions of the respective sections of the processor 4 is not kept, that is, the respective sections of the processor 4 operate with optional timings.

Next, an operation of the endoscope apparatus 1 of the present embodiment will be described.

First, a user such as a surgeon selects the white color light observation mode by operating the mode switching switch 24 in the timing before and after the power supply of the respective sections of the endoscope apparatus 1 is turned on.

When the control circuit 50 detects that the white color light observation mode is selected by the operation of the mode switching switch 24, the control circuit 50 generates a mode switching control signal corresponding to the detected result, and outputs the generated mode switching control signal to the LED driver 32, the CCD driver 48 and the light-adjusting circuit 49.

When the LED driver 32 detects that the endoscope apparatus 1 is switched to the white color light observation mode, based on the mode switching control signal which is outputted from the control circuit 50, the LED driver 32 causes the white color a light LED 31a to emit light and causes the excitation light LED 31b and the reference light LED 31c to quench lights.

When the CCD driver 48 detects that the endoscope apparatus 1 is switched to the white color light observation mode, based on the mode switching control signal which is outputted from the control circuit 50, the CCD driver 48 drives the CCD 22b, and stops drive of the CCD 23b.

Namely, by the actions and the like described above being performed, the white color light supplied from the light source apparatus 3 is emitted to the object via the light guide 7 and the illumination optical system 21, and an image of the return light (reflected light) of the white color light is formed on the image pickup surface of the CCD 22b. Subsequently, the image pickup signal obtained by the image of the return light (reflected light) of the white color light being picked up is outputted from the CCD 22b.

The image pickup signal outputted from the CCD 22b goes through the signal amplifier circuit 40 and the CDS circuit 41, and thereafter is inputted in the light-adjusting circuit 49.

When the light-adjusting circuit 49 detects that the endoscope apparatus 1 is switched to the white color light observation mode, based on the mode switching control signal which is outputted from the control circuit 50, the light-adjusting circuit 49 reads a target value WB of the brightness in the white color light observation mode from a memory or the like not illustrated. Further, based on the target value WB of the brightness read from the memory or the like not illustrated, and the image pickup signal outputted from the CDS circuit 41, the light-adjusting circuit 49 generates a light adjustment signal for adjusting the brightness of the image pickup signal, and outputs the light adjustment signal to the LED driver 32.

Subsequently, the drive electric current which is supplied from the LED driver 32 to the white color light LED 31a changes based on the light adjustment signal which is outputted from the light-adjusting circuit 49, whereby the brightness of the image pickup signal of the white color light image which is outputted from the CDS circuit 41 is adjusted so as to reach the target value WB

In the meantime, the image pickup signal outputted from the CCD 22b goes through the respective sections that are the signal amplifier circuit 40, the CDS circuit 41, the A/D converting circuit 42, the color balance adjusting circuit 43, the multiplexer 44, the synchronization memories 45a to 45c, the image processing circuit 46 and the D/A converting circuits 47a to 47c in sequence, and thereafter, the image pickup signal is outputted to the monitor 5 as a video signal.

Subsequently, the processing and the like described above are performed in the white color light observation mode, whereby the observed image (color image) corresponding to the white color light observation mode is displayed on the monitor 5.

In the meantime, before the user observes a desired observation site in an inner part of a subject, the user gives a fluorescent agent which accumulates in a lesion tissue of the desired observation site to the subject. Thereafter, the user performs an insertion operation of the endoscope 2 while watching the observed image which is displayed on the monitor 5, and thereby the user disposes a distal end portion of the endoscope 2 in a vicinity of the desired observation site inside the subject. Subsequently, in the state like this, the user or the like selects the fluorescence observation mode, by operating the mode switching switch 24.

When the control circuit 50 detects that the fluorescence observation mode is selected by the operation of the mode switching switch 24, the control circuit 50 generates a mode switching control signal corresponding to the detected result, and outputs the generated mode switching control signal to the LED driver 32, the CCD driver 48 and the light-adjusting circuit 49.

When the LED driver 32 detects that the endoscope apparatus 1 is switched to the fluorescence observation mode, based on the mode switching control signal which is outputted from the control circuit 50, the LED driver 32 causes the white color light LED 31a to quench light, causes the excitation light LED 31b and the reference light LED 31c to emit light alternately, generates identifying signals capable of identifying the time period in which the excitation light LED 31b is caused to emit light and quench light, and the time period in which the reference light LED 31c is caused to emit light and quench light respectively, and outputs the identifying signals to the timing generator 51.

When the CCD driver 48 detects that the endoscope apparatus 1 is switched to the fluorescence observation mode, based on the mode switching control signal which is outputted from the control circuit 50, the CCD driver 48 drives the CCD 23b, and stops drive of the CCD 22b.

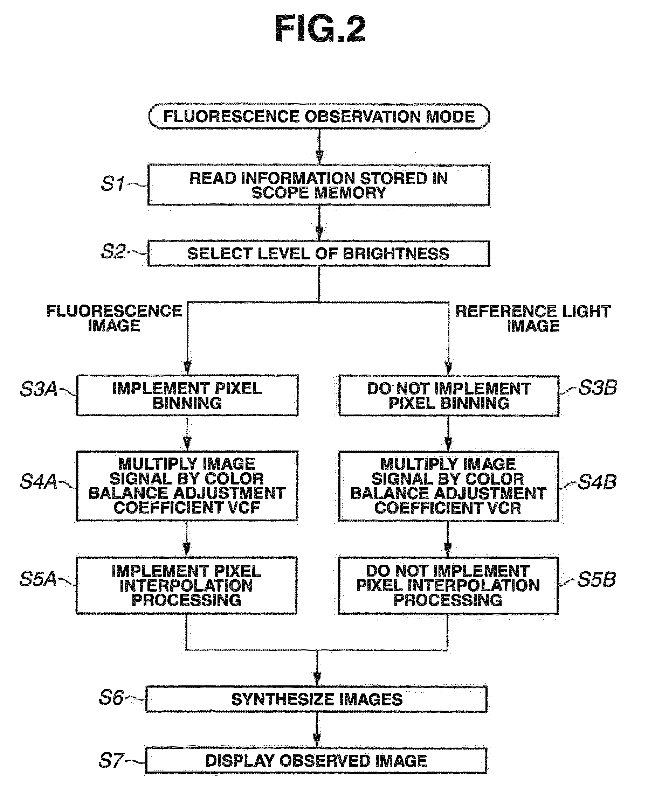

Here, description will be made with the case in which the observed image corresponding to change in brightness by the user is generated in the fluorescence observation mode cited as an example. Fig. 2 is a flowchart showing one example of the processing and the like which are performed in the fluorescence observation mode.

When the control circuit 50 detects that the fluorescence observation mode is selected by operation of the mode switching switch 24, the control circuit 50 reads the information stored in the scope memory 25 (step S1 of Fig. 2), and performs, for the image processing circuit 46, control for generating and displaying a selection screen which urges selection of one level of brightness from a level of brightness corresponding to the number of added pixels PA included in the read information, and a level of brightness corresponding to another number of added pixels which is different from the number of added pixels PA.

More specifically, on the aforementioned selection screen, the levels of the brightness corresponding to the numbers of added pixels of, for example, 2 by 2 pixels, 3 by 3 pixels, 4 by 4 pixels ... are displayed in a state in which the levels are replaced with the words of level 1, level 2, level 3, .... Further, in the aforementioned selection screen, the level of the brightness corresponding to the number of added pixels PA which is read from the scope memory 25 is selected in advance as an initial value.

When the control circuit 50 detects that one level of the brightness is selected in the aforementioned selection screen by monitoring the operation of the input apparatus group 6 (step S2 of Fig. 2), the control circuit 50 controls the CCD driver 48 to cause the CCD driver 48 to implement pixel binning by the number of added pixels PX corresponding to the one level of the brightness.

More specifically, for example, when level 1 is selected in the aforementioned selection screen, the CCD driver 48 is controlled so that pixel binning by the number of added pixels of 2 by 2 pixels.

When the CCD driver 48 detects that it is the time period in which the excitation light LED 31b emits light based on the timing signal from the timing generator 51, in the middle of the CCD driver 48 driving the CCD 23b, the CCD driver 48 acts so that pixel binning by the number of added pixels PX corresponding to the control of the control circuit 50 is implemented (step S3A of Fig. 2). Further, when the CCD driver 48 detects that it is the time period in which the reference light LED 31c emits light based on the timing signal from the timing generator 51, in the middle of the CCD driver 48 driving the CCD 23b, the CCD driver 48 acts so that pixel binning by the number of added pixels PX corresponding to the control of the control circuit 50 is not implemented (step S3B of Fig. 2).

Namely, the actions and the like described above are performed, whereby the excitation light which is supplied from the light source apparatus 3 is emitted to the object through the light guide 7 and the illumination optical system 21, in synchronization with the time period in which the excitation light LED 31b emits light, fluorescence is emanated from the fluorescence agent (given to the subject in advance) in response to irradiation of the excitation light, and the fluorescence passes through the excitation light cut filter 23 c and an image is formed on the image pickup surface of the CCD 23b. Further, the actions and the like described above are performed, whereby an electrical signal corresponding to the fluorescence an image of which is formed on the image pickup surface of the CCD 23b is generated for each of the respective pixels in synchronization with the time period in which the excitation light LED 31b emits light, and the image pickup signal with pixel binning of the number of added pixels PX being applied to the electrical signal of each of the respective pixels is outputted.

In the meantime, the action and the like described above are performed, whereby in synchronization with the time period in which the reference light LED 31c emits light, the reference light which is supplied from the light source apparatus 3 is emitted to the object through the light guide 7 and the illumination optical system 21, and an image of the return light (reflected light) of the reference light which passes through the excitation light cut filter 23c is formed on the image pickup surface of the CCD 23b. Subsequently, in synchronization with the time period in which the reference light LED 31c emits light, the image pickup signal obtained by picking up of the image of the return light (reflected light) of the reference light (image pickup signal to which pixel binning is not applied) is outputted from the CCD 23b.

Further, when the control circuit 50 detects that one level of the brightness is selected in the aforementioned selection screen, by monitoring the operation of the input apparatus group 6, the control circuit 50 controls the color balance adjusting circuit 43 so that the color balance adjusting circuit 43 performs color balance adjustment processing based on the number of added pixels PX corresponding to one level of the brightness and the target value α of the brightness ratio included in the information which is read from the scope memory 25.

The color balance adjusting circuit 43 including the function as the brightness adjusting section sets a color balance adjustment coefficient VCR which is applied (multiplied) to the image signal of the reference color image at 1, and calculates a color balance adjustment coefficient VCF which is applied (multiplied) to the image signal of the fluorescence image, as the color balance adjustment coefficients which are used in the fluorescence observation mode, based on the number of added pixels PX and the target value α which are outputted from the control circuit 50. More specifically, the color balance adjusting circuit 43 calculates a value which is obtained by the inverse number of the target value α being divided by the number of added pixels PX, for example, as the color balance adjustment coefficient VCF which is applied to the image signal of the fluorescence image. Therefore, for example, when a new number of added pixels PX is selected in the aforementioned selection screen, the value of the color balance adjustment coefficient VCF is updated (recalculated) based on the selected number of added pixels PX and the target value α.

Subsequently, the color balance adjusting circuit 43 multiplies the image signal of the fluorescence image which is inputted in synchronization with the time period in which the excitation light LED 31b emits light based on the timing signal from the timing generator 51, by the color balance adjustment coefficient VCF, and outputs the image signal to the multiplexer 44 (step S4A of Fig. 2). Further, the color balance adjusting circuit 43 multiplies the image signal of the reference light image which is inputted in synchronization with the time period in which the reference light LED 31c emits light based on the timing signal from the timing generator 51, by the color balance adjustment coefficient VCR (=1), and outputs the image signal to the multiplexer 44 (step S4B of Fig. 2).

Furthermore, when the control circuit 50 detects that one level of the brightness is selected in the aforementioned selection screen by monitoring the operation of the input apparatus group 6, the control circuit 50 controls the image processing circuit 46 to cause the image processing circuit 46 to perform pixel interpolation processing based on the number of added pixels PX corresponding to the one level of the brightness.

The image processing circuit 46 sets the number of added pixels PX which is outputted from the control circuit 50 as an interpolation scale factor SF which is used in the pixel interpolation processing of a linear interpolation method, bi-cubic interpolation method or the like. More specifically, for example, when the number of added pixels PX which is outputted from the control circuit 50 is M by M pixels, the interpolation scale factor SF is set at M²-tuple. Therefore, for example, when the new number of added pixels PX is selected in the aforementioned selection screen, the value of the interpolation scale factor SF is updated (recalculated) in accordance with the selected number of added pixels PX.

Thereafter, the image processing circuit 46 applies the pixel interpolation processing for improving a resolution by the aforementioned interpolation scale factor SF to the image signal of the fluorescence image, out of the respective image signals read from the synchronization memories 45a, 45b and 45c (step S5A of Fig. 2). On the other hand, the image processing circuit 46 does not apply the pixel interpolation processing by the interpolation scale factor SF to the image signal of the reference light image, out of the respective image signals read from the synchronization memories 45a, 45b and 45c (step S5B of Fig. 2).

The image processing circuit 46 allocates the image signal of the fluorescence image to which the pixel interpolation processing by the interpolation scale factor SF is applied and the image signal of the reference light image to which the pixel interpolation processing by the interpolation scale factor SF is not applied to the first color channel to the third color channel respectively to output the image signals to the D/A converting circuits 47a, 47b and 47c, and thereby performs image synthesis so that the fluorescence image and the reference light image are included in the same observed image (step S6 of Fig. 2). Subsequently, the observed image generated by the image synthesis processing of the image processing circuit 46 is displayed on the monitor 5 (step S7 of Fig. 2).

Namely, the pixel interpolation processing for the image signal of the fluorescence image is performed in the image processing circuit 46, whereby the resolution of the fluorescence image which is reduced in response to implementation of pixel binning is improved to the original resolution (in the case in which the pixel binning is not implemented), and the resolution of the fluorescence image and the resolution of the reference light image which are outputted to the D/A converting circuits 47a, 47b and 47c correspond to each other.

According to the present embodiment, as the pixel interpolation processing of the image processing circuit 46, for example, processing of matching a number of effective pixels of the CCD 23b and a number of displayed pixels of the monitor 5 with each other, and processing relating to improvement of the resolution by the interpolation scale factor SF may be performed in combination.

More specifically, for example, in a case in which the number of displayed pixels of the monitor 5 is twice as large as the number of effective pixels of the CCD 23b, the image processing circuit 46 may perform pixel interpolation processing by setting the interpolation scale factor of the image signal of the fluorescence image at twice as large as SF, and setting the interpolation scale factor of the image signal of the reference light image to be doubled.

The processing and the like described above are performed in the fluorescence observation mode, whereby the observed image which is obtained by image synthesis so as to include the brightness and the resolution suitable for the fluorescence image is displayed on the monitor 5, as the observed image (false-color image) corresponding to the fluorescence observation mode.

Further, according to the processing and the like described above, the value of the color balance adjustment coefficient VCF in the color balance adjusting circuit 43 is updated (recalculated) by following the change of the number of added pixels PX by the user. Therefore, according to the processing and the like described above, even when the number of added pixels PX is changed in the middle of observation by the endoscope 2, such an observed image that generates substantially no change in color tone can be displayed on the monitor 5.

Furthermore, according to the processing and the like described above, pixel binning is not performed for the reference light image. Therefore, according to the processing and the like described above, such an observed image that generates substantially no lack of useful information for diagnosis of a desired observation site can be displayed on the monitor 5.

Note that according to the processing and the like described above, the endoscope apparatus is not limited to the endoscope apparatus in which the addition processing of signals is performed in the CCD 23b having the pixel binning function, but may be the endoscope apparatus in which the addition processing of signals is performed in an addition processing circuit (not illustrated) provided between the A/D converting circuit 42 and the color balance adjusting circuit 43 of the processor 4, for example.

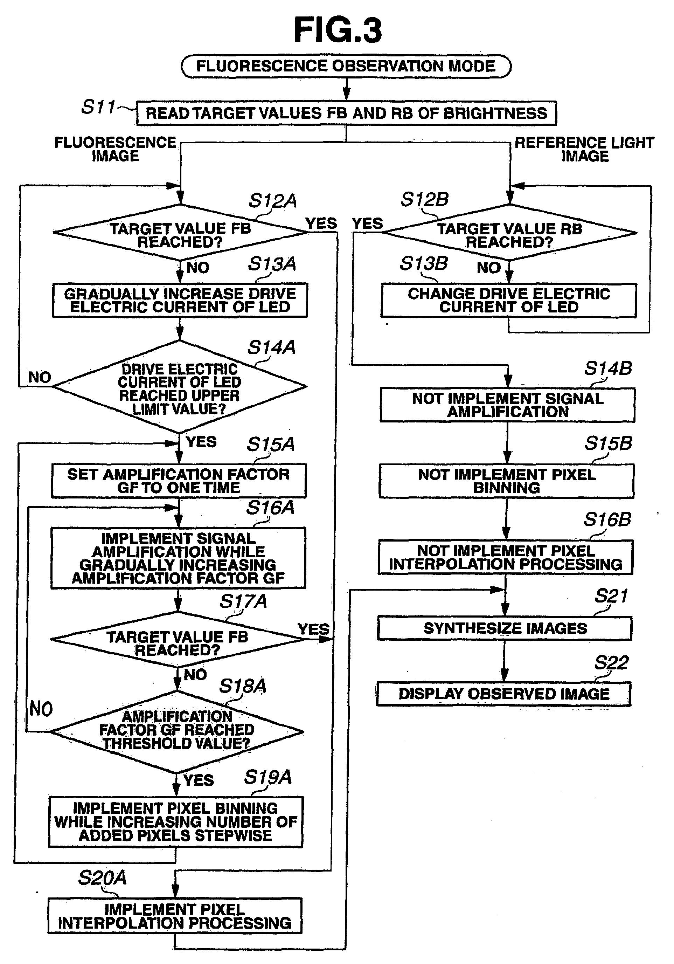

Subsequently, description will be made with the case in which an observed image corresponding to a detection result of brightness by the light-adjusting circuit 49 is generated in the fluorescence observation mode cited as an example. Fig. 3 is a flowchart showing an example of the processing and the like which are performed in the fluorescence observation mode, which is different from Fig. 2.

Note that in the following, the description will be made on the assumption that the initial value of an amplification factor GF with respect to the image pickup signal of the fluorescence image is set at one time and that the pixel binning function of the CCD 23b is off in the initial state. Further, in the following, for simplification, the detailed description will be properly omitted for the part to which the processing and the like similar to any of a series of processing illustrated in Fig. 2 are applicable.

When the light-adjusting circuit 49 detects that the endoscope apparatus 1 is switched to the fluorescence observation mode, based on the mode switching control signal outputted from the control circuit 50, the light-adjusting circuit 49 reads the target value FB of the brightness of the fluorescence image in the fluorescence observation mode, and a target value RB of the brightness of the reference light image from a memory or the like not illustrated (step S11 of Fig. 3).

Note that the target value RB is set as, for example, the brightness of the reference light image which is acquired when a reference object which includes substantially ideal reflection characteristics is irradiated with the reference light. Further, the target value FB is set as, for example, the brightness of the fluorescence image which is acquired when a reference object which includes substantially ideal fluorescence characteristics is irradiated with the excitation light. When the target values RB and FB are set according to the method like this, the value of the ratio which indicates the degree of the target value FB in the case of the value of the target value RB set as the reference, and the target value α of the aforementioned brightness ratio correspond (or substantially correspond) to each other.

Subsequently, when the light-adjusting circuit 49 detects that the brightness of the image pickup signal of the fluorescence image which is outputted from the CDS circuit 41 in synchronization with the time period in which the excitation light LED 31b emits light does not reach the target value FB, based on the timing signal from the timing generator 51 and the target value FB which is read from the memory or the like not illustrated (step S12A of Fig. 3), the light-adjusting circuit 49 generates a light adjustment signal for gradually increasing the drive electric current of the excitation light LED 31b and outputs the light adjustment signal to the LED driver 32 (step S13A and step S14A of Fig. 3).

Further, when the light-adjusting circuit 49 detects that the brightness of the image pickup signal of the reference light image which is outputted from the CDS circuit 41 in synchronization with the time period in which the reference light LED 31c emits light does not reach the target value RB, based on the timing signal from the timing generator 51 and the target value RB which is read from the memory or the like not illustrated (step S12B of Fig. 3), the light-adjusting circuit 49 generates a light adjustment signal for changing the drive electric current of the reference light LED 31c, and outputs the light adjustment signal to the LED driver 32 (step S13B of Fig. 3). Subsequently, the drive electric current which is supplied from the LED driver 32 to the reference light LED 31c changes based on the light adjustment signal which is outputted from the light-adjusting circuit 49, whereby the brightness of the image pickup signal of the reference light image which is outputted from the CDS circuit 41 is adjusted to reach the target value RB.

Also, when the light-adjusting circuit 49 which includes the function as the brightness adjusting section detects that the brightness of the image pickup signal of the fluorescence image which is outputted from the CDS circuit 41 substantially does not change while the brightness of the image pickup signal of the fluorescence image does not reach the target value FB, although the light-adjusting circuit 49 performs output of the light adjustment signal to the LED driver 32, the light-adjusting circuit 49 determines that the drive electric current which is supplied to the excitation light LED 31b reaches the upper limit value (step S14A of Fig. 3), and generates such a light adjustment signal that gradually increases the amplification factor GF to the image pickup signal of the fluorescence image from one time, and outputs the light adjustment signal to the signal amplifier circuit 40 (step S15A and step S16A of Fig. 3).

The signal amplifier circuit 40 performs signal amplification by the amplification factor GF which is increased in response to the light adjustment signal which is outputted from the light-adjusting circuit 49, for the image pickup signal of the fluorescence image which is inputted in the processor 4 in synchronization with the time period in which the excitation light LED 31b emits light, based on the timing signal from the timing generator 51, and outputs the image pickup signal of the fluorescence image to the CDS circuit 41 (step S16A of Fig. 3). Further, the signal amplifier circuit 40 does not perform signal amplification corresponding to the light adjustment signal which is outputted from the light-adjusting circuit 49 (with the amplification factor set as one time) for the image pickup signal of the reference light image which is inputted in the processor 4 in synchronization with the time period in which the reference light LED 31c emits light, based on the timing signal from the timing generator 51, and outputs the image pickup signal of the reference light image to the CDS circuit 41 (step S14B of Fig. 3).

Also, when the light-adjusting circuit 49 including the function as the brightness adjusting section detects that the amplification factor GF exceeds a threshold value TH1 (for example, quadruple) while the brightness of the image pickup signal of the fluorescence image which is outputted from the CDS circuit 41 does not reach the target value FB by monitoring the action of the signal amplifier circuit 40 while the light-adjusting circuit 49 gradually increases the amplification factor GF (step S 17A and step S18A of Fig. 3), the light-adjusting circuit 49 outputs an instruction signal aiming at performing pixel binning by setting the number of added pixels at the number of added pixels PB corresponding to the threshold value TH1 to the control circuit 50 (step S19A of Fig. 3), generates the light adjustment signal for resetting the amplification factor GF to one time, and outputs the light adjustment signal to the signal amplifier circuit 40 (step S 15A of Fig. 3). The aforementioned number of added pixels PB is set as a value which is larger than 1 by 1 pixel, like 2 by 2 pixels, for example.

Subsequently, the control circuit 50 controls the CCD driver 48 to cause the CCD driver 48 to switch the pixel binning function of the CCD 23b from off to on, and to implement pixel binning by the number of added pixels PB included in the instruction signal in response to the instruction signal from the light-adjusting circuit 49.

The light-adjusting circuit 49 generates such a light adjustment signal as gradually increases the amplification factor GF to the image pickup signal of the fluorescence image from one time, and outputs the adjustment signal to the signal amplifier circuit 40 (step S15A and step S16A of Fig. 3).

The signal amplifier circuit 40 performs signal amplification by the amplification factor GF increased in response to the light adjustment signal which is outputted from the light-adjusting circuit 49, for the image pickup signal of the fluorescence image which is inputted in the processor 4 in synchronization with the time period in which the excitation light LED 31b emits light, based on the timing signal from the timing generator 51, and outputs the light adjustment signal to the CDS circuit 41 (step S16A of Fig. 3). Further, the signal amplifier circuit 40 does not perform signal amplification corresponding to the light adjustment signal which is outputted from the light-adjusting circuit 49, for the image pickup signal of the reference light image which is inputted in the processor 4 in synchronization with the time period in which the reference light LED 31c emits light, based on the timing signal from the timing generator 51, and outputs the image pickup signal of the reference light image to the CDS circuit 41 (step S14B of Fig. 3).

Thereafter, when the light-adjusting circuit 49 detects that the amplification factor GF exceeds a threshold value TH2 (for example, 9/4-fold) while the brightness of the image pickup signal of the fluorescence image which is outputted from the CDS circuit 41 does not reach the target value FB by monitoring the action of the signal amplifier circuit 40 while the light-adjusting circuit 49 gradually increases the amplification factor GF (step S 17A and step S18A of Fig. 3), the light-adjusting circuit 49 outputs an instruction signal aiming at performing pixel binning by setting the number of added pixel at a number of added pixels PC corresponding to the threshold value TH2 to the control circuit 50 (step S19A of Fig. 3), generates the adjustment signal for resetting the amplification factor GF to one time, and outputs the light adjustment signal to the signal amplifier circuit 40 (step S15A of Fig. 3). The number of added pixels PC is set as a value that satisfies the relation of PB < PC, like 3 by 3 pixels, for example.

Subsequently, the control circuit 50 controls the CCD driver 48 to cause the CCD driver 48 to implement pixel binning by the number of added pixels PC included in the instruction signal from the light-adjusting circuit 49.

Namely, according to the light adjusting action of the light-adjusting circuit 49 as described above, in the case in which the drive electric current which is supplied to the excitation light LED 31b reaches the upper limit value, the action of gradually increasing the brightness of the image pickup signal of the fluorescence image while changing the combination of the amplification factor GF and the number of added pixels of pixel binning is repeatedly performed until the brightness of the image pickup signal reaches the target value FB. In particular, in the case in which the target values FB and RB are set by the aforementioned method, the combination of the amplification factor GF and the number of added pixels of pixel binning is repeatedly changed until the brightness ratio of the image pickup signal which is inputted in the light-adjusting circuit 49 corresponds (or substantially corresponds) to the target value α of the aforementioned brightness ratio.

Similarly to the processing illustrated in Fig. 2, in the time period in which the reference light LED 31c emits light, the control and the action for preventing pixel binning from being implemented are performed (step S15B of Fig. 3).

Here, if the assumption is made that the number of added pixels of pixel binning at the time of the brightness of the image pickup signal of the fluorescence image reaching the target value FB is PY, the image processing circuit 46 sets the number of added pixels PY as an interpolation scale factor SG which is used in pixel interpolation processing of a linear interpolation method, a bi-cubic method or the like, based on the control of the control circuit 50.

More specifically, for example, when the number of added pixels PY corresponding to the instruction signal from the light-adjusting circuit 49 is N by N pixels, the interpolation scale factor SG is set at N²-tuple. Therefore, for example, when the instruction signal aiming at performing pixel binning by setting the number of added pixels at a new number of added pixels PY is outputted to the control circuit 50 from the light-adjusting circuit 49, the value of the interpolation scale factor SG is updated (recalculated) in accordance with the number of added pixels PY.

Thereafter, the image processing circuit 46 applies the pixel interpolation processing for improving the resolution by the aforementioned interpolation scale factor SG to the image signal of the fluorescence image, out of the respective image signals which are read from the synchronization memories 45a, 45b and 45c (step S20A of Fig. 3). On the other hand, the image processing circuit 46 does not apply the pixel interpolation processing by the interpolation scale factor SG to the image signal of the reference light image, out of the respective image signals which are read from the synchronization memories 45a, 45b and 45c (step S16B of Fig. 3).

Subsequently, the image processing circuit 46 performs image synthesis so that the fluorescence image and the reference light image are included in the same observed image by allocating the image signal of the fluorescence image to which the pixel interpolation processing by the interpolation scale factor SG is applied and the image signal of the reference light image to which the pixel interpolation processing by the interpolation scale factor SG is not applied to the first color channel to the third color channel respectively, and outputting the image signals of the fluorescence image and the image signal of the reference light image to the D/A converting circuits 47a, 47b and 47c (step S21 of Fig. 3). Subsequently, the observed image generated by the image synthesis processing of the image processing circuit 46 is displayed on the monitor 5 (step S22 of Fig. 3).

Namely, the pixel interpolation processing for the image signal of the fluorescence image is performed in the image processing circuit 46, whereby the resolution of the fluorescence image which is reduced in response to implementation of pixel binning is improved to the original resolution (in the case of pixel binning being not implemented), the resolution of the fluorescence image and the resolution of the reference light image which are outputted to the D/A converting circuits 47a, 47b and 47c correspond to each other.

Note that according to the present embodiment, as the pixel interpolation processing of the image processing circuit 46, for example, the processing of matching the number of effective pixels of the CCD 23b and the number of displayed pixels of the monitor 5 with each other, and the processing relating to improvement of the resolution by the interpolation scale factor SG may be performed in combination.

More specifically, for example, in the case in which the number of displayed pixels of the monitor 5 is twice as large as the number of the effective pixels of the CCD 23b, the image processing circuit 46 may perform pixel interpolation processing by setting the interpolation scale factor of the image signal of the fluorescence image at twice as large as SG, and setting the interpolation scale factor of the image signal of the reference light image to be doubled.

The processing and the like described above are performed in the fluorescence observation mode, whereby the observed image with image synthesis performed to include the brightness and the resolution suitable for fluorescence observation is displayed on the monitor 5 as the observed image (false-color image) corresponding to the fluorescence observation mode.

Further, according to the processing and the like described above, in the signal amplifier circuit 40 disposed at the front stage of the A/D converting circuit 42, the brightness of the observed image is adjusted by amplification of the analogue image pickup signal. Therefore, according to the processing and the like as described above, the brightness of the observed image can be adjusted while occurrence of gradation jump accompanying implementation of A/D conversion is being prevented.

According to the endoscope apparatus 1 of the present embodiment, the endoscope apparatus 1 may be configured such that in the case of the fluorescence observation mode being selected, a mode in which an observed image corresponding to change of the brightness by the user is generated, and the mode in which an observed image corresponding to the detection result of the brightness by the light-adjusting circuit 49 is generated can be further selected.

The present invention is not limited to the respective embodiments described above, and various modifications and applications can be made.

The present application is filed claiming the priority of Japanese Patent Application No. 2011-119558 filed in Japan on May 27, 2011.

## Claims

1. A medical apparatus (1), comprising:
a light source section (3) capable of irradiating an object in a living body with an excitation light and a reference light alternately;
an image pickup section (23) that is configured to output a fluorescence image signal and a reference light image signal by picking up fluorescence images of fluorescence that is emanated when a fluorescent substance existing in the object is excited by irradiation of the excitation light, and by picking up reference light images of reflected light that is the reference light reflected in the object, respectively;
a storage section (25) that is configured to store in advance information including a target value of a brightness ratio of the fluorescence image and the reference light image that are picked up by the image pickup section (23),
a pixel addition processing section (23b) that is configured to perform pixel binning processing of making a signal corresponding to a number of added pixels in the fluorescence image signal obtained by picking up of the fluorescence image of the fluorescence be a signal corresponding to one pixel;
a brightness adjusting section (43) that is configured to perform adjustment of brightness so that a brightness ratio of the fluorescence image to which the pixel addition processing is applied and the reference light image obtained by picking up of an image of the reflected light has a constant value,
wherein the brightness adjusting section (43) is configured to calculate a color balance adjustment coefficient based on the target value stored in the storage section (25), and is configured to perform adjustment of brightness by using the calculated color balance adjustment coefficient,
wherein, when the number of added pixels in the pixel binning processing is changed, the brightness adjusting section (43) is configured to update the color balance adjustment coefficient based on the changed number of added pixels and the target value, and is configured to perform adjustment of brightness by using the updated color balance adjustment coefficient; and
an image processing section (46) that is configured to apply pixel interpolation processing to the fluorescence image signal to which the adjustment of brightness is applied in the brightness adjusting section (43), and to perform image synthesis so that the fluorescence image signal to which the pixel interpolation processing is applied and the reference light image signal to which the adjustment of brightness is applied in the brightness adjusting section (43) are included in a same observed image.

2. The medical apparatus (1) according to claim 1,
wherein the image processing section (46) is configured to perform pixel interpolation processing by setting an interpolation scale factor based on the number of added pixels in the pixel binning processing, for causing a resolution of the fluorescence image signal to which the adjustment of brightness is applied in the brightness adjusting section (43) to correspond to a resolution of the reference light image signal to which the adjustment of brightness is applied in the brightness adjusting section (43),
wherein, when the number of added pixels in the pixel binning processing is changed, the image processing section (46) is configured to update the interpolation scale factor based on the changed number of added pixels..

3. The medical apparatus (1) according to claim 1, wherein the brightness adjusting section (43) is configured to calculate a value obtained by dividing an inverse number of the target value by the number of added pixels as the color balance adjustment coefficient.

4. The medical apparatus (1) according to claim 1,
wherein the image pickup section (23) has a function of the pixel addition processing section (23b), and the pixel binning processing is performed in an image pickup device (23b) provided in the image pickup section (23).

## Patentansprüche

1. Medizinisches Gerät (1), das umfasst:
einen Lichtquellenabschnitt (3), der dazu in der Lage ist, ein Objekt in einem lebenden Körper abwechselnd mit einem Anregungslicht und einem Referenzlicht zu bestrahlen;
einen Bildaufnahmeabschnitt (23), der dazu eingerichtet ist, ein Fluoreszenzbildsignal und ein Referenzlichtbildsignal auszugeben, indem er Fluoreszenzbilder von Fluoreszenz aufnimmt, die ausgestrahlt wird, wenn eine in dem Objekt existierende fluoreszente Substanz durch die Bestrahlung mit dem Anregungslicht angeregt wird, bzw. indem er Referenzlichtbilder von reflektiertem Licht aufnimmt, das das in dem Objekt reflektierte Referenzlicht ist;
einen Speicherabschnitt (25), der dazu eingerichtet ist, vorab eine Information zu speichern, die einen Zielwert eines Helligkeitsverhältnisses des Fluoreszenzbilds und des Referenzlichtbilds, die von dem Bildaufnahmeabschnitt (23) aufgenommen werden, umfasst,
einen Pixeladditionsverarbeitungsabschnitt (23b), der dazu eingerichtet ist, eine Pixelbinningverarbeitung durchzuführen, bei der ein Signal, das einer Anzahl von addierten Pixeln in dem durch Aufnehmen des Fluoreszenzbilds der Fluoreszenz erhaltenen Fluoreszenzbildsignal entspricht, zu einem Signal gemacht wird, das einem Pixel entspricht;
einen Helligkeitsanpassungsabschnitt (43), der dazu eingerichtet ist, eine Helligkeitsanpassung vorzunehmen, so dass ein Helligkeitsverhältnis des Fluoreszenzbilds, auf das die Pixeladditionsverarbeitung angewendet wird, und des Referenzlichtbilds, das durch Aufnehmen eines Bilds des reflektierten Lichts erhalten wird, einen konstanten Wert hat,
wobei der Helligkeitsanpassungsabschnitt (43) dazu eingerichtet ist, auf der Basis des in dem Speicherabschnitt (25) gespeicherten Zielwerts einen Farbausgleichsanpassungskoeffizienten zu berechnen und dazu eingerichtet ist, unter Verwendung des berechneten Farbausgleichsanpassungskoeffizienten eine Helligkeitsanpassung vorzunehmen,
wobei, wenn die Anzahl von addierten Pixeln in der Pixelbinningverarbeitung verändert wird, der Helligkeitsanpassungsabschnitt (43) dazu eingerichtet ist, den Farbausgleichsanpassungskoeffizienten auf der Basis der veränderten Anzahl von addierten Pixeln und des Zielwerts zu aktualisieren und dazu eingerichtet ist, unter Verwendung des aktualisierten Farbausgleichsanpassungskoeffizienten eine Helligkeitsanpassung vorzunehmen; und
einen Bildverarbeitungsabschnitt (46), der dazu eingerichtet ist, eine Pixelinterpolationsverarbeitung auf das Fluoreszenzbildsignal anzuwenden, auf das die Helligkeitsanpassung in dem Helligkeitsanpassungsabschnitt (43) angewendet wird, und eine Bildsynthese durchzuführen, so dass das Fluoreszenzbildsignal, auf das die Pixelinterpolationsverarbeitung angewendet wird, und das Referenzlichtbildsignal, auf das die Helligkeitsanpassung in dem Helligkeitsanpassungsabschnitt (43) angewendet wird, in dem gleichen beobachteten Bild enthalten sind.

2. Medizinisches Gerät (1) gemäß Anspruch 1,
wobei der Bildverarbeitungsabschnitt (46) dazu eingerichtet ist, eine Pixelinterpolationsverarbeitung durchzuführen, indem er einen Interpolationsskalierungsfaktor auf der Basis der Anzahl von addierten Pixeln in der Pixelbinningverarbeitung festlegt, um zu veranlassen, dass eine Auflösung des Fluoreszenzbildsignals, auf das die Helligkeitsanpassung in dem Helligkeitsanpassungsabschnitt (43) angewendet wird, einer Auflösung des Referenzlichtbildsignals entspricht, auf das die Helligkeitsanpassung in dem Helligkeitsanpassungsabschnitt (43) angewendet wird,
wobei, wenn die Anzahl von addierten Pixeln in der Pixelbinningverarbeitung verändert wird, der Bildverarbeitungsabschnitt (46) dazu eingerichtet ist, den Interpolationsskalierungsfaktor auf der Basis der veränderten Anzahl von Pixeln zu aktualisieren.

3. Medizinisches Gerät (1) gemäß Anspruch 1, bei dem der Helligkeitsanpassungsabschnitt (43) dazu eingerichtet ist, einen Wert, der durch Dividieren einer inversen Zahl des Zielwerts durch die Anzahl von addierten Pixeln erhalten wird, als den Farbausgleichsanpassungskoeffizienten zu berechnen.

4. Medizinisches Gerät (1) gemäß Anspruch 1,
wobei der Bildaufnahmeabschnitt (23) eine Funktion des Pixeladditionsverarbeitungsabschnitts (23b) hat und die Pixelbinningverarbeitung in einer in dem Bildaufnahmeabschnitt (23) vorgesehenen Bildaufnahmeeinrichtung (23b) durchgeführt wird.

## Revendications

1. Appareil médical (1), comprenant :
une section de source de lumière (3) capable d'irradier un objet dans un corps vivant avec une lumière d'excitation et une lumière de référence alternativement ;
une section de capture d'image (23) qui est configurée pour délivrer en sortie un signal d'image de fluorescence et un signal d'image de lumière de référence en capturant des images de fluorescence de la fluorescence qui est émanée lorsqu'une substance fluorescente se trouvant dans l'objet est excitée par l'irradiation de la lumière d'excitation, et en capturant des images de lumière de référence de la lumière réfléchie qui est la lumière de référence réfléchie dans l'objet, respectivement ;
une section de mémoire (25) qui est configurée pour mémoriser à l'avance des informations contenant une valeur cible d'un rapport de luminosité de l'image de fluorescence et de l'image de lumière de référence qui sont capturées par la section de capture d'image (23),
une section de traitement d'addition de pixels (23b) qui est configurée pour réaliser un traitement de classification de pixels consistant à amener un signal correspondant à un nombre de pixels additionnés dans le signal d'image de fluorescence obtenu en capturant l'image de fluorescence de la fluorescence à être un signal correspondant à un pixel ;
une section d'ajustement de luminosité (43) qui est configurée pour réaliser un ajustement de la luminosité de sorte qu'un rapport de luminosité de l'image de fluorescence auquel le traitement d'addition de pixels est appliqué et de l'image de lumière de référence obtenue en capturant une image de la lumière réfléchie présente une valeur constante,
dans lequel la section d'ajustement de luminosité (43) est configurée pour calculer un coefficient d'ajustement de l'équilibre des couleurs sur la base de la valeur cible mémorisée dans la section de mémoire (25), et est configurée pour réaliser un ajustement de la luminosité en utilisant le coefficient d'ajustement de l'équilibre des couleurs calculé,
dans lequel, lorsque le nombre de pixels additionnés dans le traitement de classification de pixels est changé, la section d'ajustement de luminosité (43) est configurée pour mettre à jour le coefficient d'ajustement de l'équilibre des couleurs sur la base du nombre de pixels additionnés changé et de la valeur cible, et est configurée pour réaliser un ajustement de la luminosité en utilisant le coefficient d'ajustement de l'équilibre des couleurs mis à jour ; et
une section de traitement d'image (46) qui est configurée pour appliquer un traitement d'interpolation de pixel au signal d'image de fluorescence auquel l'ajustement de luminosité est appliqué dans la section d'ajustement de luminosité (43), et pour réaliser une synthèse d'image de sorte que le signal d'image de fluorescence auquel le traitement d'interpolation de pixel est appliqué et le signal d'image de lumière de référence auquel l'ajustement de luminosité est appliqué dans la section d'ajustement de luminosité (43) sont contenus dans une même image observée.

2. Appareil médical (1) selon la revendication 1,
dans lequel la section de traitement d'image (46) est configurée pour réaliser un traitement d'interpolation de pixel en établissant un facteur d'échelle d'interpolation sur la base du nombre de pixels additionnés dans le traitement de classification de pixels, pour amener une résolution du signal d'image de fluorescence auquel l'ajustement de luminosité est appliqué dans la section d'ajustement de luminosité (43) à correspondre à une résolution du signal d'image de lumière de référence auquel l'ajustement de luminosité est appliqué dans la section d'ajustement de luminosité (43),
dans lequel, lorsque le nombre de pixels additionnés dans le traitement de classification de pixels est changé, la section de traitement d'image (46) est configurée pour mettre à jour le facteur d'échelle d'interpolation sur la base du nombre de pixels additionnés changé.

3. Appareil médical (1) selon la revendication 1, dans lequel la section d'ajustement de luminosité (43) est configurée pour calculer une valeur obtenue en divisant un nombre inverse de la valeur cible par le nombre de pixels additionnés en tant que coefficient d'ajustement de l'équilibre des couleurs.

4. Appareil médical (1) selon la revendication 1,
dans lequel la section de capture d'image (23) possède une fonction de la section de traitement d'addition de pixels (23b), et le traitement de classification de pixels est réalisé dans un dispositif de capture d'image (23b) prévu dans la section de capture d'image (23).
